Europäisches Patentamt

European Patent Office

Office européen des brevets

(19)

(11) **EP 0 734 454 B1**

(12) **EUROPÄISCHE PATENTSCHRIFT**

(45) Veröffentlichungstag und Bekanntmachung des Hinweises auf die Patenterteilung:
**26.09.2001 Patentblatt 2001/39**

(21) Anmeldenummer: **95920765.5**

(22) Anmeldetag: **31.05.1995**

(51) Int Cl.[7]: **C12Q 1/26**, G01N 27/327

(86) Internationale Anmeldenummer:
**PCT/DE95/00741**

(87) Internationale Veröffentlichungsnummer:
**WO 95/33071 (07.12.1995 Gazette 1995/52)**

(54) **VERFAHREN UND ELEKTROCHEMISCH-ENZYMATISCHES INDIKATIONSSYSTEM ZUR BESTIMMUNG VON NITRIT**

ELECTROCHEMICAL ENZYMATIC INDICATOR SYSTEM AND METHOD USING THIS SYSTEM FOR THE DETERMINATION OF NITRITE

PROCEDE ET SYSTEME D'INDICATION ELECTROCHIMICO-ENZYMATIQUE POUR LA DETERMINATION DE LA PRESENCE DE NITRITE

(84) Benannte Vertragsstaaten:
**AT CH DE FR GB IT LI NL SE**

(30) Priorität: **31.05.1994 DE 4420391**

(43) Veröffentlichungstag der Anmeldung:
**02.10.1996 Patentblatt 1996/40**

(73) Patentinhaber:
• **UFZ-UMWELTFORSCHUNGSZENTRUM LEIPZIG-HALLE GMBH**
**04318 Leipzig (DE)**
• **PRIVATE UNIVERSITÄT WITTEN/HERDECKE GMBH**
**58448 Witten (DE)**

(72) Erfinder:
• **SCHUMACHER, Wolfram**
**D-78464 Konstanz (DE)**
• **KRONECK, Peter, M., H.**
**D-78467 Konstanz (DE)**
• **BARTHOLMES, Peter**
**D-58456 Witten (DE)**
• **KOTTE, Heiner**
**D-04277 Leipzig (DE)**
• **STREHLITZ, Beate**
**D-04129 Leipzig (DE)**

(74) Vertreter: **Gulde, Klaus W., Dipl.-Chem. et al**
**Patentanwälte**
**Gulde Hengelhaupt Ziebig**
**Schützenstrasse 15-17**
**10117 Berlin (DE)**

(56) Entgegenhaltungen:
• **CHEMICAL ABSTRACTS, Band 118, Nr. 17, ver!ffentlicht 26. April 1993 (Columbus, Ohio, USA) C. MORENO et al. "Electro- chemical studies of the hexahemenitrite reductase from Desulfoyibrio desulfuri- cans ATCC 27774", Seite 375, Nr. 163 972n; & Eur.J.Biochem. 1993, 212(1), 79-86.**
• **CHEMICAL ABSTRACTS, Band 121, Nr. 23, ver!ffentlicht 05. Dezember 1994 (Columbus, Ohio, USA) B.STREHLITZ et al. "Artifi- cial electron donors for nitrate and nitrite reducta- ses usable as mediators in amperometric biosensors" Seite 520, Nr. 275 629z; & Fresenius J. Anal.Chem. 1994, 349(8-9), 676-8.**
• **CHEMICAL ABSTRACTS, Band 84, Nr. 11, ver!ffentlicht 15. M rz 1976 (Columbus, Ohio, USA) C.-H. KIANG et al. "A novel enzyme electrode method for the determination of nitrite based on nitrite reductase" Seite 188, Nr. 71 108m; & Anal.Chim.Acta 1975, 80(2), 209-14**

**Beschreibung**

In der Routineanalytik wird Nitrit häufig durch eine spektrometrische Methode bestimmt, wobei Sulfanilamid in saurer Lösung durch Nitritionen diazotiert und anschließend durch Kupplung mit N-(1-Naphthyl)-ethylendiamin zu einem roten Farbstoff umgesetzt wird. Die bei 540 nm gemessene spektrale Absorption steht in linearer Beziehung zur Nitritkonzentration (DIN EN 26777, 1993 bzw. DIN 38 405 Teil 10, 1981; DIN 38 405 Teil 28,1993; Johannssen et al. DE 4004900, 1990). Das verwendete N-(1-Naphthyl)ethylendiamin ist kanzerogen. Weitere bekannte spektrometrische Verfahren benutzen die Kinetik der Entfärbung des Eisen(III)-Thiocyanat - Komplexes, die von der Nitritkonzentration abhängig ist (Utsumi et al., Bull. of the Chem. Soc. of Japan, 51, 3496,1978), bzw. von Thionin, dessen Oxidation durch Kaliumbromat von Nitrit katalysiert wird (Jiang et al., Anal. Chim. Acta 234, 403, 1990).

Nachteil aller spektrometrischer Verfahren sind Störungen durch die Eigenfärbung der Probe, der Umgang mit toxischen Reagenzien, deren Entsorgung sowie der vergleichsweise hohe Zeitbedarf zur Durchführung der Analyse.

Eine weitere Standardmethode ist die Nitritbestimmung mittels Ionenchromatographie (DIN 38 405 Teil 19, 1988), die jedoch einen großen gerätetechnischen Aufwand und entsprechendes Fachpersonal erfordert. Eine Vor-Ort-Bestimmung ist auf diese Weise nicht möglich. Die Kosten pro Analyse sind relativ hoch.

Zu den elektrochemischen Methoden gehört die Nitritbestimmung über seine Reduktion bzw. Oxidation. Eine sehr empfindliche Methode stellt die Verwendung einer Kohlenstoffpasteelektrode in Verbindung mit einem Ionenaustauscher dar (Kaicher, Talanta, 33, 489, 1986). Nitrit wird auf diese Weise auf der Elektrodenoberfläche angereichert und dann mittels Differential-Puls-.Polarographie anodisch bestimmt. Interferenzen durch andere Ionen wie beispielsweise $Br^-$, $NO_3^-$, $Cl^-$, $I^-$ sind sehr groß und können bis zu 100 % des Nitritsignals betragen. Holak und Specchio (Anal. Chem. 64,1313,1992) nutzen die Differential-Puls-Polarographie für den Nachweis des aus der Reduktion von Nitrit mit Natriumbromid zu NO und anschließender Spülung mit Stickstoff aus Diphenylamin gebildeten Diphenylnitrosamins an einer Quecksilbertropfelektrode. Das gebildete Diphenylnitrosamin ist giftig. Sulaiman ( Anal. Chem. 56, 1984, 2405) bestimmt Nitrit mittels Differential-Puls-Polarographie das Diazotierungsprodukt von Sulfanilsäure bzw. Orthanilsäure durch Nitrit. Die Methode wird durch andere Ionen gestört.

Für eine amperometrische Nachweismethode zur Nitritbestimmung wird eine Kohlefasermikroelektrode mit dem Polymer $[Os(bipy)_2(PVP)_{20}Cl]Cl$ beschichtet (Malone et al., Analyst, 117,1259,1992). Diese Elektrode wird in einer Fließ-Injektions-Analyse mit einem sauren Trägerstrom verwendet, der die Bildung von $NO^+$ aus $NO_2^-$ ermöglicht. Die Redoxreaktion $NO^+ + Os^{II}$ --> $NO(gasf.) + Os^{III}$ wird bei - 150 mV gemessen und ist ein Maß für die Nitritkonzentration. Da diese Methode durch Fe(III) gestört wird, müssen die Proben zur Entfernung oder Komplexierung von Fe(III)-Ionen vorbehandelt werden.

Die enzymatische Nitritbestimmung wurde erstmals von Kiang et al. (Anal. Chim. Acta 80, 209, 1975) beschrieben. Sie benutzten die aus Spinat isolierte Nitritreduktase (EC 1.6.6.4) zur Reduktion des Nitrit zu Ammonium, das sie mit einer potentiometrischen Gaselektrode auf Basis einer pH-Elektrode nachwiesen. Die Nachteile liegen in der geringen Empfindlichkeit der potentiometrischen Indikation, es treten hohe Querempfindlichkeiten auf und es sind definierte Ionenkonzentrationen im Meßmedium nötig.

Die bekannten Methoden der Nitritbestimmung haben den Nachteil einer mangelnden Selektivität bzw. Sensivität bzw. können diesen Nachteil nur durch einen großen gerätetechnischen bzw. personellen Aufwand vermeiden. Giftige Sustanzen, die für diese Nachweisreaktioneen oft erforderlich sind, müssen entsorgt werden.

Moreno, C. et al. beschreiben in Eur.J. Biochem., Vol. 212, No.1 (1993):79-86 cyclisch-voltammetrische und chronoamperometrische kinetische Studien zwischen drei verschiedenen Mediatoren, nämlich dem "künstlichen" Mediator Methylviologen, dem natürlichen Mediator Cytochrom $C_3$ aus D. desulfuricans und aus D. vulgaris (als Elektronendonator), und Nitritreduktase aus *Desulfovibrio desulfuricans*. Die verwendete Arbeits-Elektrode ist aus Glas, als Referenzelektrode fungiert eine Ag/AgCl-Elektrode.

Der Erfindung lag nun die Aufgabe zugrunde, ein alternatives Verfahren zur Bestimmung von Nitrit sowie ein elektrochemisch-enzymatisches Indikationssystem bereitzustellen.

Das Verfahren zur Bestimmung von Nitrit in wäßrigen Medien oder zum Nachweis nitritbildender Reaktionen durch ein elektrochemisch-enzymatisches Indikationssystem unter Verwendung einer amperometrischen Redoxelektrode, eines Elektronendonators und einer Nitrit zu Ammonium reduzierenden und sechs Elektronen übertragenden Nitritreduktase, vorzugsweise für Biosensoren und Teststreifen, wird erfindungsgemäß dadurch gelöst, daß die Oberfläche der amperometrischen Redoxelektrode mit der Nitritreduktase aus *Sulfurospirillum deleyianum* und dem Elektronendonator Phenosafranin in Kontakt gebracht wird und der katodische Reduktionsstrom des Elektronendonators, der der Nitritkonzentration direkt proportional ist, gemessen wird.

Das erfindungsgemäße Verfahren gewährleistet durch seine effiziente Elektronentransferreaktion eine sehr gute Empfindlichkeit, ist schnell, kostengünstig und ermöglicht ohne toxische Reagenzien die quantitative Messung von Nitrit auch in gefärbten, trüben oder feststoffhaltigen wäßrigen Medien. Das Meßsystem ist sehr gut in Form kostengünstiger planarer Einwegsensoren herstellbar. Es erfordert keine oder nur eine geringe Probenvorbereitung und ist insbesondere zur Vor-Ort-Analyse geeignet.

Das amperometrische Stromsignal ist der Analytkonzentration direkt proportional. Der in der Trinkwasserversorgung festgeschriebene Grenzwert (0,1 mg/l) liegt im linearen Meßbereich.

Das Meßsystem ist einfach bedienbar und ermöglicht auch die Anwendung durch Hilfskräfte.

Besonders vorteilhaft ist es, als amperometrische Redoxelektrode eine Kohlenstoffelektrode oder Edelmetallelektrode, die eine Polarisationsspannung zwischen -100 mV und -800 mV (vs. SCE) aufweist, zu verwenden.

Weiterhin ist es vorteilhaft, daß die Nitritreduktase in gelöster oder immobilisierter Form verwendet wird.

Eine weitere vorteilhafte Gestaltung des erfindungsgemäßen Verfahrens besteht darin, den Elektronendonator in gelöster Form oder als schwer wasserlösliche Komponente in das Elektrodenmaterial einzubringen oder kovalent am Enzym oder an der Elektrodenoberfläche gebunden einzusetzen.

Das erfindungsgemäße Verfahren zur elektrochemischenzymatischen Nitritbestimmung wird nach den in den Gleichungen 1.a -1.b oder 2.a -2.c angegebenen enzymatischen und amperometrischen reaktionsschritten besonders vorteilhaft ausgeführt.

Redoxelektrode

$$6 \; Med_{ox} \quad + \quad 6 \; e^- \; - - - - - - - - - \; 6 \quad Med_{red} \qquad\qquad 1.a$$

$$NO_2^- \; + \; 8 \; H^+ \; + \; 6 \quad Med_{red} \; - - - - - - - - \; NH_4^+ \; + \; 6 \; Med_{ox} \; + \; 2 \; H_2O \qquad 1.b$$

Redoxelektrode

$$6 \; Med \; (1)_{ox} \; + 6 \; e^- \; - - - - - - - - - \; 6 \quad Med \; (1)_{red} \qquad\qquad 2.a$$

$$6 \; Med \; (1)_{red} \; + \; 6 \; Med \; (2)_{ox} \; - - - - - \; 6 \quad Med \; (2)_{red} \; + \; 6 \; Med \; (1)_{ox} \qquad 2.b$$

$$NO_2^- \; + \; 8 \; H^+ \; + \; 6 \; Med \; (2)_{red} \; - - - - - \; NH_4^+ \; + \; 6 \; Med \; (2)_{ox} \; + \; 2 \; H_2O \qquad 2.c$$

Das erfindungsgemäße elektrochemisch-enzymatische Indikationssystem zur Bestimmung von Nitrit in wäßrigen Medien oder zum Nachweis nitritbildender Reaktionen besteht aus dem Elektronendonator Phenosafranin, der in gelöster oder immobilisierter Form verwendeten Nitritreduktase aus *Sulfurospirillum deleyianum* und einer an sich bekannten amperometrischen Redoxelektrode, wobei der Elektronendonator in gelöster Form oder als schwer wasserlösliche Komponente in das Elektrodenmaterial eingebracht oder kovalent am Enzym oder der Elektrodenoberfläche gebunden verwendet wird und wobei der katodische Reduktionsstrom des Elektronendonators als Maß für die Nitritreduktion dient.

Beispiel 1:

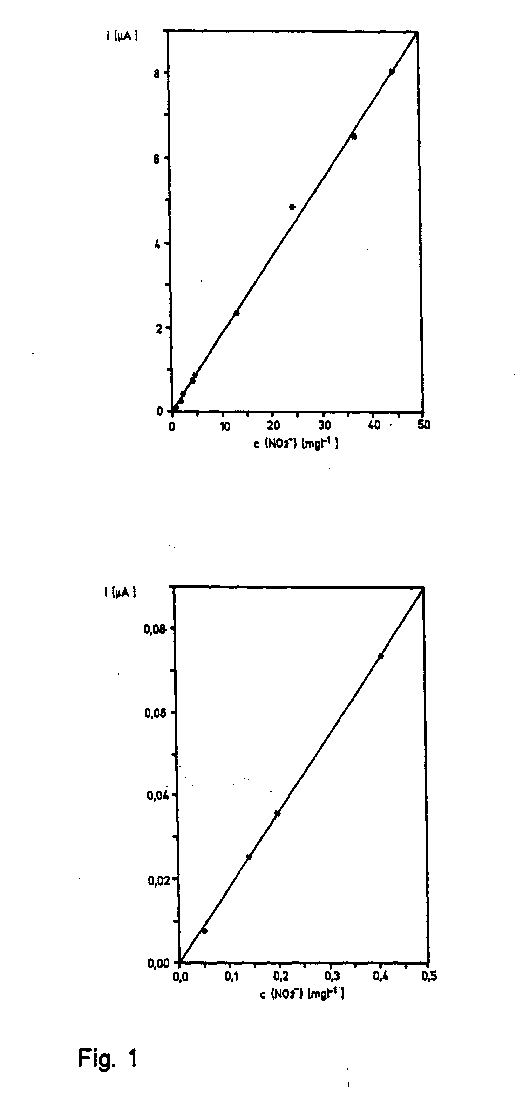

Auf der Oberfläche einer mediatormodifizierten Elektrode (Graphitelektrode mit inkorporiertem, durch Anionenaustausch mittels Tetraphenylborat schwer wasserlöslich gemachtem Phenosafranin) wurde mittels einer Membran Cuprophan 80 M (AKZO Wuppertal) durch dip-coating aufgebrachte Nitritreduktase aus *Sulfurospirillum deleyianum* (51 U/cm$^2$) nativ fixiert. Zur Messung wurde die Elektrode in 0,125 M Phosphatpufferlösung pH 7,4, die ständig mit Stickstoff gespült wurde, verwendet und auf das für die Reduktion des redoxmediators Phenosafranin spezifische Potential von -600 mV gegen eine Kalomelelektrode unter Verwendung einer Platin-Gegenelektrode polarisiert. Die Messung des katalytischen Stromes nach Zugabe unterschiedlicher Nitritkonzentrationen ergab die in Figur 1 dargestellte Kalibrierkurve.

Beispiel 2:

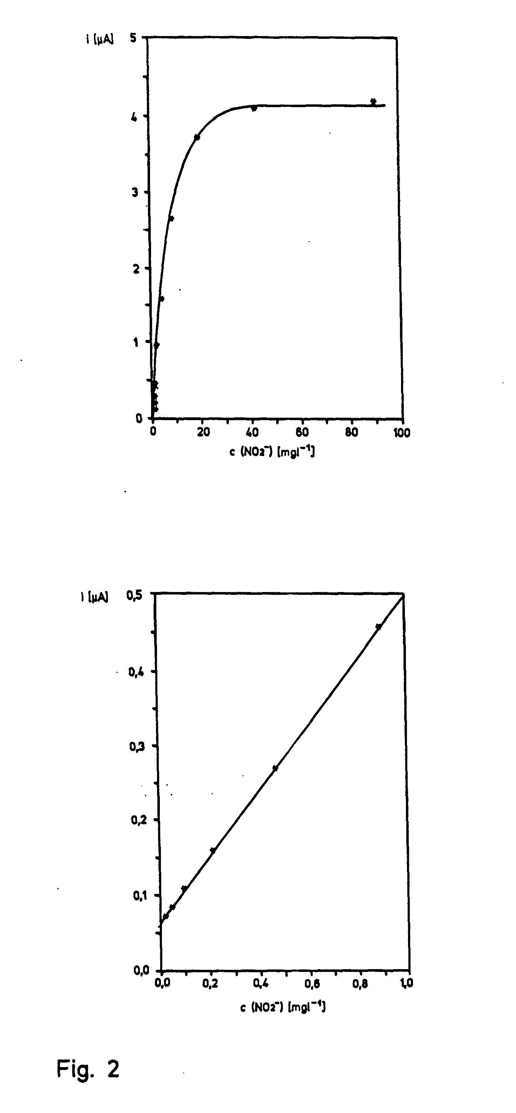

Nitritreduktase aus Sulfurospirillum deleyianum wurde mittels Glutaraldehydvernetzung an Rinderserumalbumin auf einer Membran Cuprophan 80 M (AKZO Wuppertal) immobilisiert. Diese Immobilisierung wurde auf der Oberfläche einer Graphitelektrode mittels Spannring gefestigt, so daß die Enzymkonzentration auf der Elektrodenoberfläche 1,77 U/cm$^2$ betrug. Die Elektrode wurde zur Messung in 0,125 M Phosphatpufferlösung pH 7,4, die 1 mmol/ l Mediator Safranin T enthielt und ständig mit Stickstoff gespült wurde, verwendet. Sie wurde auf das für die Reduktion des Redoxmediators spezifische Potential von -600 mV gegen eine Kalomelelektrode unter Verwendung einer Platin - Gegenelektrode polarisiert. Nach der Zugabe unterschiedlicher Nitritkonzentrationen wurde der katalytische Strom gemessen. Diese Kalibrierkurve ist in Figur 2 dargestellt.

**Patentansprüche**

1. Verfahren zur Bestimmung von Nitrit in wäßrigen Medien oder zum Nachweis nitritbildender Reaktionen unter Verwendung einer amperometrischen Redoxelektrode, eines Elektronendonors und einer Nitrit zu Ammonium reduzierenden und sechs Elektronen übertragenden Nitritreduktase,
   **dadurch gekennzeichnet, daß**
   die Oberfläche der amperometrischen Redoxelektrode mit der Nitritreduktase aus *Sulfurospirillum deleyianum* und dem Elektronendonor Phenosafranin in Kontakt gebracht wird und der katodische Reduktionsstrom des Elektronendonors, der der Nitritkonzentration direkt proportional ist, gemessen wird.

2. Verfahren nach Anspruch 1,
   **dadurch gekennzeichnet, daß**
   als amperometrische Redoxelektrode eine Kohlenstoffelektrode oder Edelmetallelektrode, die eine Polarisationsspannung zwischen -100 mV und -800 mV (vs. SCE) aufweist, dient.

3. Verfahren nach Anspruch 1 oder 2,
   **dadurch gekennzeichnet, daß**
   die Nitritreduktase in gelöster oder immobilisierter Form verwendet wird.

4. Verfahren nach den Ansprüchen 1 bis 3,
   **dadurch gekennzeichnet, daß** der Elektronendonor in gelöster Form oder als schwer wasserlösliche Komponente in das Elektrodenmaterial eingebracht oder kovalent am Enzym oder an der Elektrodenoberfläche gebunden verwendet wird.

5. Elektrochemisch-enzymatisches Indikationssystem zur Bestimmung von Nitrit in wäßrigen Medien oder zum Nachweis nitritbildender Reaktionen, bestehend aus dem Elektronendonor Phenosafranin, der in gelöster oder immobilisierter Form verwendeten Nitritreduktase aus *Sulfurospirillum deleyianum* und einer an sich bekannten amperometrischen Redoxelektrode, wobei das Phenosafranin in gelöster Form oder als schwer wasserlösliche Komponente in das Elektrodenmaterial eingebracht oder kovalent am Enzym oder der Elektrodenoberfläche gebunden verwendet wird und der katodische Reduktionsstrom des Elektronendonors das Maß für die Nitritreduktion ist.

**Claims**

1. A method of determining nitrite in aqueous media or of detecting nitrite-forming reactions using an amperometric redox electrode, an electron donor and a nitrite reductase reducing nitrite to form ammonium and transferring six electrons,
   **characterized in that**
   the surface of the amperometric redox electrode is contacted with nitrite reductase from *Sulfurospirillum deleyianum* and phenosafranine electron donor, and the cathodic reduction current of the electron donor is measured, which current is directly proportional to the nitrite concentration.

2. The method according to claim 1,
   **characterized in that**
   a carbon electrode or a noble metal electrode having a polarization voltage of between -100 mV and -800 mV (vs. SCE) is used as amperometric redox electrode.

3. The method according to claim 1 or 2,
**characterized in that**
the nitrite reductase is employed in dissolved or immobilized form.

4. The method according to any of claims 1 to 3,
**characterized in that**
the electron donor is incorporated in the electrode material in dissolved form or as a component sparingly soluble in water, or is employed covalently bound to the enzyme or to the electrode surface.

5. An electrochemical-enzymatic indication system to determine nitrite in aqueous media or detect nitrite-forming reactions, consisting of said phenosafranine electron donor, said nitrite reductase from *Sulfurospirillum deleyianum* used in dissolved or immobilized form, and a *per se* known amperometric redox electrode, said phenosafranine being incorporated in the electrode material in dissolved form or as a component sparingly soluble in water or employed covalently bound to the enzyme or to the electrode surface, and the cathodic reduction current of the electron donor being a measure of nitrite reduction.


**Revendications**

1. Procédé de détermination du nitrite en milieu aqueux ou de mise en évidence de réactions formant des nitrites en utilisant une électrode rédox ampérométrique, un donneur d'électrons et une réductase de nitrite transférant six électrons et réduisant un nitrite en ammonium,
**caractérisé en ce que**
l'on met en contact la surface de l'électrode rédox ampérométrique avec la réductase de nitrite de *Sulfurospirillum deleyianum* et le donneur d'électrons phénosafranine et que l'on mesure le courant de réduction cathodique du donneur d'électrons qui est directement proportionnel à la concentration en nitrite.

2. Procédé d'après la revendication 1,
**caractérisé en ce que**
l'on utilise, comme électrode rédox ampérométrique, une électrode en carbone ou en métal noble qui présente une tension de polarisation comprise entre -100 mV et -800 mV (vis à vis de la SCE).

3. Procédé d'après la revendication 1 ou 2,
**caractérisé en ce que**
la réductase de nitrite est utilisée sous forme solubilisée ou forme immobilisée.

4. Procédé d'après les revendications 1 à 3,
**caractérisé en ce que**
le donneur d'électrons est utilisé en l'appliquant dans le matériau de l'électrode sous une forme solubilisée ou sous forme de composant peu soluble dans l'eau, ou en le liant de manière covalente à l'enzyme ou à la surface de l'électrode.

5. Système d'indication enzymatique/électrochimique pour déterminer le nitrite en milieu aqueux et pour mettre en évidence les réactions de formation de nitrite, composé du donneur d'électrons phénosafranine, de la réductase de nitrite de *Sulfurospirillum deleyianum* utilisée sous forme solubilisée ou immobilisée, et d'une électrode rédox ampérométrique connue, dans lequel la phénosafranine est utilisée en l'appliquant dans la matériau de l'électrode sous une forme solubilisée ou en tant que composant peu soluble dans l'eau ou en la liant de manière covalente à l'enzyme ou à la surface de l'électrode, et dans lequel le courant de réduction cathodique du donneur d'électrons sert de grandeur pour mesurer la réduction de nitrite.

Fig. 1

Fig. 2